# EUROPÄISCHE PATENTANMELDUNG

(11) **EP 2 149 384 A1**
(43) Veröffentlichungstag der Anmeldung: **03.02.2010**
(21) Anmeldenummer: 09164635.6
(22) Anmeldetag: 06.07.2009
(51) Int. Cl.: A61M 5/50

(54) **Spritze**

(30) Priorität: 02.08.2008 DE 102008036202
(71) Anmelder: Meddrop Technology AG, 9001 St Gallen (SE)
(72) Erfinder: Gulik, Dieter c/o MedDrop AG, 9000, St. Gallen (CH); Aulebach, Henner, 22045, Hamburg (DE)
(74) Vertreter: Meyer, Ludgerus

(57) **Zusammenfassung**

Die Erfindung betrifft eine Spritze zur Verabreichung einer Dosis vom im Spritzenkörper (1) der Spritze aufgenommener Flüssigkeit über eine an den Spritzenkörper (1) ansetzbare Spritzennadel, wobei der Spritzenkörper (1) hohlzylindrisch ausgebildet ist, und ein Spritzenkolben (2) mittels rückseitig auf den Spritzenkolben aufgebrachtem Druck im Hohlraum des Spritzenkörpers (1) vorschiebbar ist. Rückseitig des Spritzenkolbens (2) ist eine Federscheibe (4) mit rückwärtig schräg zur Innenwand des Spritzenkörpers gerichteten Zähnen vorgesehen, welche beim Vortrieb des Spritzenkolbens auf der Innenwandung des Spritzenkörpers (1) gleitet und bei einem Zurückdrücken des Spritzenkolbens durch Verkeilen mit der Innenwandung des Spritzenkörpers (1) ein Zurückziehen der Federscheibe (4) und des Spritzenkolbens (2) aus jeder Stellung verhindert.

## Beschreibung

### Technisches Gebiet

Die Erfindung betrifft eine Spritze zur Verabreichung einer Dosis von im Spritzenkörper der Spritze aufgenommener Flüssigkeit nach dem Oberbegriff des Anspruchs 1.

Übliche Spritzen bestehen aus einem Spritzenkörper, der hohlzylindrisch ausgebildet ist, wobei im Hohlraum des Spritzenkörpers mittels eines rückseitig betätigbaren Stößels ein Spritzenkolben vorschiebbar ist. Die im Spritzenkörper enthaltene Flüssigkeit wird über eine an den Spritzenkörper ansetzbare Spritzennadel durch rückwärtigen Druck auf den Kolben ausgestoßen.

Zur Erhöhung der Sicherheit bei der Benutzung einer Spritze sind eine Vielzahl von Spritzenvariationen bekannt geworden. Im Wesentlichen betreffen diese Variationen Maßnahmen, um die Nadel der Spritze nach der Benutzung zu sichern oder zu zerstören. Heute werden auch vielfach Einwegspritzen verwendet, die nach der Benutzung entsorgt werden.

In einigen Anwendungsfällen ist es allerdings auch heute noch üblich, Spritzenkörper mehrfach zu verwenden, insbesondere weil entweder Einwegspritzen nicht zur Verfügung stehen, das zu verabreichende Medikament nur in größeren Gebinden vorrätig ist, aus denen die Flüssigkeit in den Spritzenkörper zunächst eingefüllt werden muss, oder auch im Bereich der Tiermedizin, in dem häufig große Mengen an Flüssigkeiten zu verabreichen sind, welches entsprechend großvolumige Spritzenkörper erfordert, die aus Ersparnisgründen häufig wiederverwendet werden.

In vielen Fällen ist es aber gewünscht, Einwegspritzen zu verwenden, insbesondere um zu vermeiden, dass Anwender bereits benutzte Spritzen verwenden, um andere Flüssigkeiten in den Spritzenkörper aufzuziehen und dann damit Risiken zu verursachen.

### Stand der Technik

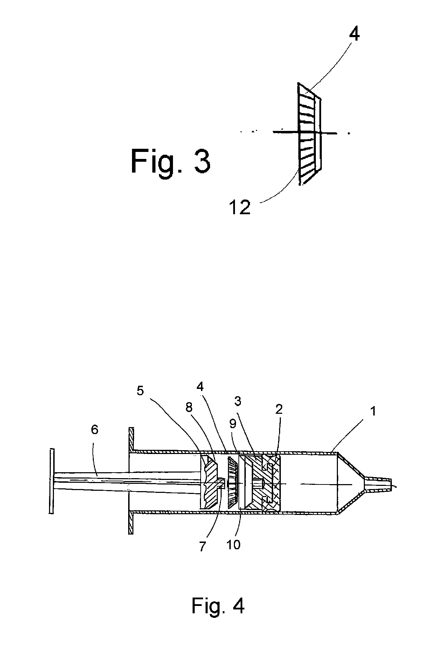

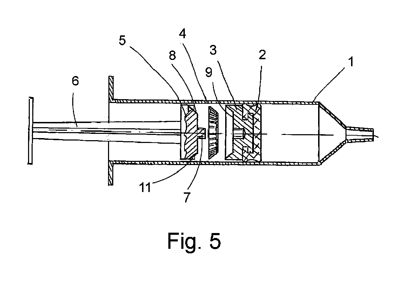

Aus der US 5,106,372 ist eine Spritze bekannt, bei der sich eine mit einem Ausschnitt versehene leicht gebogene Scheibe zwischen Druckkolben und Spritzenkolben befindet, die beim Vorschieben des Spritzenkolbens bis zum distalen Ende hinter eine innenseitig im Spritzenkörper befindliche ringförmige Verdickung treten kann, so dass die Verdickung es beim Zurückziehen des Spritzenkolbens verhindert, dass die Scheibe über die ringförmige Verdickung gezogen werden kann. Damit wird ein Zurückziehen des Spritzenkolbens aus seiner distalen Stellung verhindert. Die entsprechenden Zustände des Spritzenkolbens im Spritzenkörper und der Rückhaltescheibe ergeben sich insbesondere aus den Figuren 4 und 5 der Entgegenhaltung, wobei Fig. 4 den Zustand unmittelbar vor Erreichen der ringförmigen Verdickung (24) zeigt, während Fig. 5 den Zustand zeigt, in dem die Scheibe (62) hinter die Verdickung (24) getreten ist.

### Darstellung der Erfindung

Der Erfindung liegt daher die Aufgabe zugrunde, eine Spritze anzugeben, die nur zum einmaligen Gebrauch verwendet werden kann, wobei es möglich sein soll, ein Zurückziehen des Spritzenkolbens aus dem Spritzenkörper in jeder Lage zu verhindern.

Diese Aufgabe wird durch die im Anspruch 1 angegebene Erfindung gelöst. Vorteilhafte Weiterbildungen der Erfindung sind in Unteransprüchen angegeben.

Eine erfindungsgemäße Spritze enthält einen Spritzenkörper, an den eine Spritzennadel ansetzbar ist. Der Spritzenkörper ist hohlzylindrisch ausgebildet. Mittels eines rückseitig aufbringbaren Druckes ist ein Spritzenkolben betätigbar, und damit kann die im Spritzenkörper aufgenommene Flüssigkeit über die Nadel abgegeben werden. Der Druck auf den Spritzenkolben kann über einen Stößel erfolgen oder über Druckluft oder hydraulisch aufgebracht werden. An der Rückseite des Spritzenkolbens befindet sich eine Federscheibe, die einen nach außen schräg zur Innenwand des Spritzenkörpers gerichteten Rand aufweist, der in der Lage ist, ein Zurückziehen der Federscheibe und des Spritzenkörpers zu verhindern. Erfindungsgemäß ist die Federscheibe mit rückwärtig schräg zur Innenwandung des Spritzenkörpers gerichteten und beim Vortrieb des Spritzenkolbens auf der Innenwandung des Spritzenkörpers gleitenden Zähnen versehen. Bei einem Zurückdrücken oder Zurückziehen des Spritzenkolbens verkeilen sie sich jedoch mit der Innenwandung des Spritzenkörpers, so dass ein Zurückziehen der Federscheibe und damit des Spritzenkolbens aus jeder Stellung des Spritzenkolbens verhindert wird.

Bei einer Ausführungsform, bei der der Druck über einen Stößel aufgebracht wird, der an seinem vorderen Ende einen Druckkolben enthält, welcher beim Vortrieb auf die Rückseite des Spritzenkolbens einwirkt, ist die Federscheibe zwischen Spritzenkolben und Druckkolben angeordnet.

Der Stößel kann auch über einen Durchbruch der Federscheibe unmittelbar am Spritzenkolben befestigt sein, wobei die Federscheibe an der Rückseite des Spritzenkolbens befestigt ist, z. B. über rückseitige Ansätze des Spritzenkolbens, die durch die Federscheibe greifen oder über eine Verbreiterung des Stößels, die die axiale Verschiebung der Federscheibe verhindert.

Die Spritze nach der Erfindung kann in üblicher Weise betätigt werden, indem z. B. der Stößel nach vorne geschoben wird und damit die Flüssigkeit im Spritzenkörper ausgestoßen wird. Wenn jedoch nach Ende der Verabreichung oder während der Verabreichung versucht wird, den Kolben zurückzuziehen, wird dies durch die Federscheibe verhindert, die sich beim Zurückziehen des Kolbens mit der Innenwandung des Spritzenkörpers verkeilt. Dadurch kann der Kolben nur in eine Richtung bis zum Ende vorgeschoben werden, und es besteht keine Möglichkeit, die Spritze erneut zu verwenden, indem z. B. versucht wird, eine neue Dosis an Flüssigkeit über die Nadel in den Spritzenkörper aufzuziehen.

Die bei der Erfindung verwendete Federscheibe, die insbesondere aus Edelstahl gebildet ist, ist scheibenförmig gestaltet und weist außenseitig durch radiale keilförmige Einschnitte gebildete Zähne auf, die im spitzen Winkel leicht nach hinten in Achsrichtung abgebogen sind. Beim Vorschieben der Federscheibe gleiten die Zähne daher an der Innenwandung des Spritzenkörpers, während beim Zurückziehen sich die Zähne an der Innenwand des Spritzenkörpers festhaken, da die Innenwand gegenüber dem Material der Federscheibe aus weichem Kunststoff besteht, so dass die Zähne der Federscheibe sich ohne weiteres im Kunststoff verankern können.

Im flachen Zustand ist die Federscheibe im Durchmesser etwas größer als der Innendurchmesser des Spritzkörpers, so dass sich die Zähne der Federscheibe beim Einschieben in den Spritzenkörper nach hinten biegen.

Die Federscheibe wird bei Verwendung eines Druckkolbens vorzugsweise passend zwischen dem Druckkolben und dem Spritzenkolben eingesetzt, indem der Druckkörper eine vordere Oberfläche und/oder der Spritzenkolben eine rückwärtige Oberfläche haben, die der Form der Federscheibe entsprechen.

In einer weitergehenden Ausführungsform kann der Spritzenkolben einen rückseitigen äußeren zylindrischen Ansatz aufweisen, in dem die Federscheibe beim Vorschieben des Stößels ohne Berührung mit der Innenwandung des Spritzenkörpers aufgenommen ist. Bei der Abgabe der Flüssigkeit im Spritzenkörper steht die Federscheibe daher nicht in unmittelbarer Berührung mit der Innenwand des Spritzenkörpers, sondern ruht im zylindrischen Ansatz des Spritzenkolbens. Erst beim Zurückziehen des Stößels und des mit ihm unmittelbar verbundenen Druckkolbens sowie der daran befestigten Federscheibe löst sich die Federscheibe aus dem zylindrischen Ansatz und kann in die Innenwandung des Spritzenkörpers eingreifen. Voraussetzung dafür ist, dass Stößel, Druckkolben und Federscheibe keine feste Einheit bilden, sondern einen freien Bewegungsweg zueinander haben, der so lang ist, dass die Federscheibe beim Zurückziehen des Stößels aus dem Ansatz des Spritzenkolbens heraustreten kann.

Der Vorteil dieser Ausbildungsform liegt darin, dass eine Befüllung des Spritzenkörpers von der Nadelanschlussseite her möglich bleibt, da bei vorderseitigem Druck auf den Spritzenkolben die Federscheibe im Bereich des zylinderförmigen Ansatzes des Spritzenkolbens verbleibt, so dass ein Zurückschieben des Spritzenkolbens mit Federscheibe und Druckkolben und Stößel möglich ist.

In einer alternativen weiteren Ausbildungsform kann anstelle des Spritzenkolbens der Druckkolben einen vorderseitigen zylindrischen Ansatz aufweisen, in dem beim Vorschieben des Druckkolbens die Federscheibe aufgenommen ist. Wenn die Federscheibe mit dem Spritzenkolben verbunden ist, und der Druckkolben gegenüber dem Spritzenkolben um eine gewisse Strecke verschieblich bleibt, tritt die Federscheibe beim Zurückziehen des Druckkolbens aus dessen Ansatz heraus und verhindert damit das Zurückziehen des Spritzenkolbens.

Aufgrund der Bewegungsmöglichkeit des Stößels gegenüber dem Spritzenkolben bei den vorgenannten Optionen ist es von Vorteil, wenn der Stößel am hinteren Ende des Spritzenkörpers eine seitliche Führung erfährt.

### Kurze Beschreibung der Abbildungen der Zeichnungen

Die Erfindung wird nachstehend anhand eines Ausführungsbeispiels näher erläutert. Es zeigen:

Fig. 1 eine Schnittansicht durch eine erfindungsgemäße Spritze,

Fig. 2 eine Aufsicht auf eine verwendete Federscheibe,

Fig. 3 eine Seitenansicht einer Federscheibe,

Fig. 4 eine erste alternative Ausführungsform einer erfindungsgemäßen Spritze, und

Fig. 5 eine zweite Alternative einer erfindungsgemäßen Spritze.

### Darstellung eines Ausführungsbeispiels

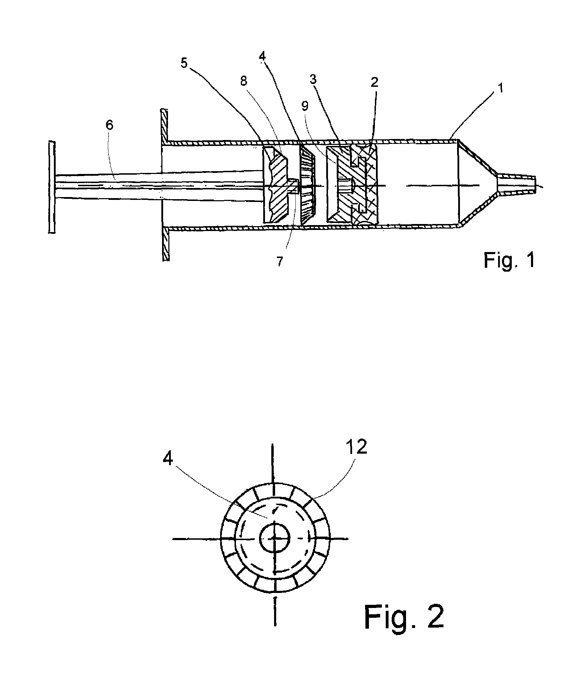

Die in Fig. 1 dargestellte Spritze enthält einen Spritzenkörper 1, der hohlzylindrisch ausgebildet ist und an seinem vorderen Ende eine Tülle zum Ansatz einer Spritzennadel aufweist. Im Spritzenkörper 1 ist ein Spritzenkolben 2 verschieblich gelagert, der aus einem vorderen abdichtenden Gummi oder Kunststoff und einem rückwärtigen Halter 3 besteht, an dem der eigentliche Spritzenkolben 2 über rückseitige Ansätze befestigt ist. Der Halter 3 des Spritzenkolbens weist an der Rückseite 9 eine Ausnehmung auf, in die die Federscheibe 4 eintreten kann. Auf der Rückseite der Federscheibe 4 befindet sich der Druckkolben 5, dessen Vorderseite 8 der Rückseite der Federscheibe 4 angepasst ist. Der Druckkolben 5 ist mit dem Stößel 6 einstückig verbunden. Am vorderen Ende des Druckkolbens 5 befindet sich ein Ansatz 7, der durch eine Öffnung der Federscheibe 4 hindurchgeführt ist und über eine Schraubverbindung mit dem Halter 3 des Druckkolbens verschraubbar ist.

Die Einheit, gebildet aus Spritzenkolben 2, Halter 3, Federscheibe 4, Druckkolben 5 und Stößel 6 lässt sich durch Druck auf die Rückseite des Stößels 6 vorwärtsbewegen, so dass ein im Spritzenkörper aufgenommenes Flüssigkeitsvolumen durch die Tülle des Spritzenkörpers abgegeben werden kann.

Die Federscheibe 4 ist scheibenförmig ausgebildet und weist im ebenen Zustand einen etwas größeren Durchmesser als der Innendurchmesser des Spritzenkörpers auf. Beim Einsetzen in den Spritzenkolben werden die Zähne leicht nach hinten gebogen. Die Federscheibe gleitet beim Vorschieben des Druckkolbens daher entlang der Innenwandung des Spritzenkörpers. Aufgrund der unter Federkraft stehenden Außenzähne der Federscheibe 4 ist nur eine Bewegung der Federscheibe in eine Richtung zum vorderen Ende der Spritze hin möglich. Ein Zurückziehen wird durch die Zähne der Federscheibe verhindert, die beim Zurückziehen in die Seitenwandung des Spritzenkörpers eingreifen.

Fig. 2 zeigt eine Federscheibe in Aufsicht. Die insbesondere aus Edelstahl bestehende Scheibe weist äußere radial gerichtete keilförmig Einschnitte auf. Die auf diese Weise gebildeten Zahnlappen sind in Axialrichtung mit einem spitzen Winkel abgebogen, so dass sie bei Einsetzen in den Zylinderkörper eine Art Kalotte bilden, die im Spritzenkörper nur in eine Richtung bewegbar ist.

Fig. 3 zeigt die Federscheibe in Seitenansicht mit den nach hinten abgebogenen Zähnen.

Fig. 4 zeigt eine alternative Ausführungsform, bei der der Halter 3 des Spritzenkolbens einen rückwärtigen äußeren zylindrischen Ansatz 10 aufweist, in den die Federscheibe 4 einschiebbar ist. Dabei ist die Federscheibe 4 fest mit dem Druckkolben 5 verbunden. Der Achsansatz 7 ist zwar fest mit dem Spritzenkolben 2 verbunden, jedoch bei dieser Ausführung axial um eine Strecke beweglich, die etwa der Länge des Ansatzes 10 entspricht. Während das Vorschieben des Spritzenkolbens in gleicher Weise wie mit der Ausführungsform nach Fig. 1 erfolgt, wobei jedoch die Federscheibe nicht an der Innenwandung des Spritzenkörpers gleitet, sondern im Ansatz 10 aufgenommen ist, führt das Zurückziehen des Druckkolbens 5 mit daran befestigter Federscheibe dazu, dass die Zähne der Federscheibe sich aus dem Ansatz 10 lösen und dann in die Innenwandung des Spritzenkörpers eingreifen können. Solange sich die Zähne der Federscheibe an der Innenseite des Ansatzes 10 befinden, kann der Spritzenkolben 2 auch in rückwärtiger Richtung bewegt werden, was insbesondere die Möglichkeit schafft, zur Befüllung des Spritzenkörpers Flüssigkeitsdruck über die Tülle des Spritzenkörpers aufzubringen. Damit lässt sich eine automatische Befüllung derartiger Spritzenkörper ermöglichen. Ein manuelles Aufziehen von Flüssigkeit wird jedoch gleichwohl verhindert. Um zu verhindern, dass sich die Zähne im Ansatz verankern, muss dieser entweder sehr hart ausgebildet sein oder die Federscheibe darf nur ein sehr kleines Stück in den Ansatz geschoben sein, so dass sie leicht aus dem Ansatz herausgezogen werden kann.

Fig. 5 zeigt eine alternative Ausbildung zu Fig. 4, bei der der zylindrische Ansatz 11 sich an der Vorderseite des Druckkörpers 5 befindet. Solange die Zähne der Federscheibe 4 sich im Bereich des Ansatzes 11 befinden, lässt sich durch vorderseitigen Druck auf den Druckkolben 2 ein Zurückschieben der Kolben ermöglichen. Wenn jedoch der Stößel 6 zurückgezogen wird, treten die Zähne der sich bei dieser Ausführungsform an dem Halter 3 befestigten Federscheibe aus dem Ansatz 11 heraus und können sich an der Innenseite des Spritzenkörpers verkrallen. Auch bei dieser Ausführungsform wird daher ein manuelles Zurückziehen der Kolben verhindert, während zur Befüllung des Spritzenkörpers eine Druckeinführung von Flüssigkeit über die Tülle möglich bleibt.

Die zuletzt dargestellte Ausführungsform hat den weiteren Vorteil, dass bereits nach einmaligem Zurückziehen des Stößels 6 keine Möglichkeit des erneuten Zurückziehens besteht, auch wenn Druck von der Vorderseite des Spritzenkörpers in den Spritzenkörper eingebracht wird, da die Zähne der Federscheibe 4 nicht wieder in den Bereich des Ansatzes 11 bringbar sind. Bei der Ausführungsform nach Fig. 4 können die Zähne jedoch bei erneutem Eindrücken der Federscheibe in den Ansatz 10 wieder "freigegeben werden".

Für den Fall, dass auf den Druckkolben verzichtet wird und der Stößel unmittelbar am Spritzenkolben verankert oder daran sonst wie befestigt ist, ist sicherzustellen, dass die Federscheibe beim Vorschieben des Spritzenkolbens daran verankert bleibt. Dies kann z. B. durch Ansätze des Kunststoff-Spritzenkolbens erfolgen, die durch Öffnungen der Federscheibe hindurchgeführt sind und auf der Rückseite thermisch verbreitert werden. Andere Befestigungsmöglichkeiten, wie Verschraubungen oder Klemmung zwischen Spritzenkolben und Stößel sind ebenfalls denkbar.

Bei hydraulischer oder pneumatischer Betätigung des Spritzenkolbens kann u. U. auch auf eine Befestigung der Federscheibe verzichtet werden, wenn sichergestellt ist, dass die Rückseite der Federscheibe gegenüber den Innenquerschnitt des Spritzenkolbens abgedichtet ist, damit der hydraulische oder pneumatische Druck den Spritzenkolben zusammen mit der Federscheibe als Einheit bewegt. Bei Verwendung eines separaten Druckstößels, der nicht mit dem Spritzenkolben verbunden ist, der in die Rückseite des Spritzenkolbens eingeführt wird und Druck auf die Federscheibe und den Spritzenkolben ausübt, kann auch auf die Abdichtung verzichtet werden.

Die Erfindung bietet die Möglichkeit, die erneute Verwendung einer Einmalspritze sicher zu verhindern. Der Aufbau ist einfach und die Spritze kann kostengünstig hergestellt werden.

### Bezugszeichenliste

1 Spritzenkörper

2 Spritzenkolben

3 Halter

4 Federscheibe

5 Druckkolben

6 Stößel

7 Achsansatz

8 Vorderseite

9 Rückseite

10 Ansatz

11 Ansatz

12 Einschnitte

## Patentansprüche

1. Spritze zur Verabreichung einer Dosis von im Spritzenkörper (1) der Spritze aufgenommener Flüssigkeit über eine an den Spritzenkörper (1) ansetzbare Spritzennadel, wobei der Spritzenkörper (1) hohlzylindrisch ausgebildet ist, und ein Spritzenkolben (2) mittels rückseitig auf den Spritzenkolben aufgebrachtem Druck im Hohlraum des Spritzenkörpers (1) vorschiebbar ist, wobei rückseitig des Spritzenkolbens (2) eine Federscheibe (4) mit rückwärtig schräg zur Innenwand des Spritzenkörpers gerichtetem Außenrand vorgesehen ist, welcher in der Lage ist, ein Zurückziehen der Federscheibe (4) und des Spritzenkolbens (2) zu verhindern, **dadurch gekennzeichnet, dass** die Federscheibe (4) mit rückwärtig schräg zur Innenwand des Spritzenkörpers gerichteten und beim Vorschieben des Spritzenkolbens auf der Innenwand des Spritzenkörpers gleitenden Zähnen versehen ist, welche beim Zurückdrücken des Spritzenkolbens durch Verkeilen mit der Innenwandung des Spritzenkörpers (1) ein Zurückdrücken der Federscheibe (4) und des Spritzenkolbens aus jeder Stellung des Spritzenkolbens im Spritzenkörper verhindert.

2. Spritze nach Anspruch 1, **dadurch gekennzeichnet, dass** die Federscheibe (4) an der Rückseite des Spritzenkolbens (2) befestigt ist.

3. Spritze nach Anspruch 2, **dadurch gekennzeichnet, dass** die Befestigung über rückseitige Ansätze des Spritzenkolbens erfolgt, die durch Öffnungen der Federscheibe hindurchgreifen.

4. Spritze nach Anspruch 1, **dadurch gekennzeichnet, dass** der Spritzenkolben (2) mittels eines rückseitig betätigbaren Stößels (6) im Hohlraum des Spritzenkörpers (1) vorschiebbar ist, dass der Stößel (6) an seinem vorderen Ende einen Druckkolben (5) aufweist, welcher beim Vortrieb auf die Rückseite des Spritzenkolbens (2) einwirkt, und dass die Federscheibe zwischen Spritzenkolben (2) und Druckkolben (5) angeordnet ist.

5. Spritze nach Anspruch 4, **dadurch gekennzeichnet, dass** die Rückseite (9) des Spritzenkolbens eine Ausnehmung aufweist, die der Form der Federscheibe (4) im Wesentlichen folgt.

6. Spritze nach Anspruch 4, **dadurch gekennzeichnet, dass** der Druckkolben (5) eine Vorderseite (8) aufweist, die der Form der Federscheibe (4) im Wesentlichen folgt.

7. Spritze nach Anspruch 5, **dadurch gekennzeichnet, dass** der Spritzenkolben einen rückseitigen äußeren zylindrischen Ansatz (10) aufweist, in dem die Federscheibe beim Vorschieben des Stößels (6) ohne Berührung mit der Innenwandung des Spritzenkörpers aufgenommen ist.

8. Spritze nach Anspruch 6, **dadurch gekennzeichnet, dass** der Druckkolben einen vorderseitigen äußeren zylindrischen Ansatz (11) aufweist, in dem die Federscheibe (4) beim Vorschieben des Stößels ohne Berührung mit der Innenwandung des Spritzenkörpers (1) aufgenommen ist.

9. Spritze nach Anspruch 4, 5 oder 6, **dadurch gekennzeichnet, dass** der Kolben über einen die Federscheibe durchdringenden Achsansatz (7) des Stößels (6) gekoppelt sind.

10. Spritze nach Anspruch 8 und 9, **dadurch gekennzeichnet, dass** der Achsansatz (7) mit einem Spiel solcher Länge im Spritzenkolben (2) verankert ist, dass beim Zurückziehen des Stößels (6) die Zähne der Federscheibe (4) aus dem zylindrischen Ansatz heraustreten können, wobei die Federscheibe (4) mit der Rückseite des Spritzenkolbens verbunden ist.

11. Spritze nach Anspruch 7 und 9, **dadurch gekennzeichnet, dass** der Achsansatz (7) mit einem Spiel solcher Länge im Spritzenkolben (2) verankert ist, dass beim Zurückziehen des Stößels die Zähne der Federscheibe aus dem zylindrischen Ansatz (11) heraustreten können, wobei die Federscheibe (4) mit der Vorderseite des Druckkolbens (5) verbunden ist.

12. Spritze nach einem oder mehreren der vorhergehenden Ansprüche, **dadurch gekennzeichnet, dass** die Federscheibe als metallische kreisförmige Scheibe (4) mit äußeren radialen keilförmigen Einschnitten (12) ausgebildet ist, und dass die durch die Einschnitte (12) gebildeten Federelemente in Axialrichtung im spitzen Winkel von der Ebene der Federscheibe abgebogen sind.

13. Spritze nach Anspruch 12, **dadurch gekennzeichnet, dass** die Federscheibe (4) aus Edelstahl besteht.
